# EUROPÄISCHE PATENTSCHRIFT

(11) **EP 0 613 882 B1**
(45) Veröffentlichungstag und Bekanntmachung des Hinweises auf die Patenterteilung: **08.01.1997**
(21) Anmeldenummer: 94102861.5
(22) Anmeldetag: 25.02.1994
(51) Int. Cl.: C07C 253/30, C07C 255/19

(54) **Verfahren zur Herstellung von 5-Cyanvaleriansäureestern**
Process for the preparation of 5-cyanovaleric acid ester
Procédé de préparation d'esters de l'acide 5-cyanovalérique

(30) Priorität: 03.03.1993 DE 4306507
(43) Veröffentlichungstag der Anmeldung: 07.09.1994
(73) Patentinhaber: BASF Aktiengesellschaft, 67063 Ludwigshafen (DE)
(72) Erfinder: Stahl, Stefan, Dr., D-67549 Worms (DE); Harder, Wolfgang, Dr., D-69469 Weinheim (DE); Hoehn, Arthur, Dr., D-67063 Ludwigshafen (DE)

(56) Entgegenhaltungen:
- EP-A- 0 373 579
- EP-A- 0 377 838
- DE-A- 2 541 640
- GB-A- 1 497 046

## Beschreibung

Die vorliegende Erfindung betrifft ein verbessertes Verfahren zur Herstellung von 5-Cyanvaleriansäureestern (I) durch Carbonylierung eines 1-Cyanbutens mit Kohlenmonoxid und einem dem Esterrest entsprechenden Alkanol (II) bei erhöhter Temperatur und erhöhtem Druck in Gegenwart eines Cobaltkatalysators sowie in Gegenwart eines aktivierenden Lösungsmittels.

Nach der allgemeinen Lehre der EP-A 373 579 ist es bekannt, 1-Cyanbutene durch Carbonylierung mit Kohlenmonoxid und Wasser oder einem Alkanol bei erhöhtem Druck und erhöhter Temperatur mit Hilfe von Cobaltkatalysatoren in die 5-Cyanvaleriansäure bzw. in die Alkylester dieser Sauren zu überführen, wobei diese Umsetzung in Gegenwart von 70 bis 99 Gew.-%, bezogen auf die Menge des Reaktionsgemisches, eines Lactams oder eines cyclischen Harnstoffderivates als aktivierendem Lösungsmittel vorgenommen wird. Soweit es die Herstellung der Ester betrifft, ist diese Reaktion lediglich für den Fall des Methylesters unter Verwendung des Lactams N-Methylpyrrolidon beschrieben, jedoch ist hier der Umsatz von 12,9 % unbefriedigend.

Der Erfindung lag daher die Aufgabe zugrunde, die Alkylester der 5-Cyanvaleriansäure besser zuganglich zu machen als bisher. Insbesondere waren aktivierende Lösungsmittel gesucht, die auch bei relativ geringer Konzentration eine vorteilhafte Wirkung zeigen.

Demgemäß wurde ein verbessertes Verfahren zur Herstellung von 5-Cyanvaleriansäureestern (I) durch Carbonylierung eines 1-Cyanbutens mit Kohlenmonoxid und einem dem Esterrest entsprechenden Alkanol (II) bei erhöhter Temperatur und erhöhtem Druck in Gegenwart eines Cobaltkatalysators sowie in Gegenwart eines aktivierenden Lösungsmittels gefunden, welches dadurch gekennzeichnet ist, daß man als aktivierendes Lösungsmittel wirksame Mengen eines Kohlensäurediesters, eines Carbaminsäureesters oder eines Harnstoffes der allgemeinen Formeln IIIa, IIIb bzw. IIIc

R-O-CO-O-R (IIIa)

R₂N-CO-O-R (IIIb)

R₂N-CO-NR₂ (IIIc)

oder von Gemischen dieser Verbindungen verwendet, in denen die Reste R gleich oder verschieden sein können und für C₁- bis C₆-Alkylgruppen oder C₅- bis C₇-Cycloalkylgruppen stehen, wobei diese Reste auch unter Ausbildung 5- bis 7-gliedriger Ringe miteinander verbunden sein können.

Die Reaktion läßt sich wie folgt veranschaulichen: 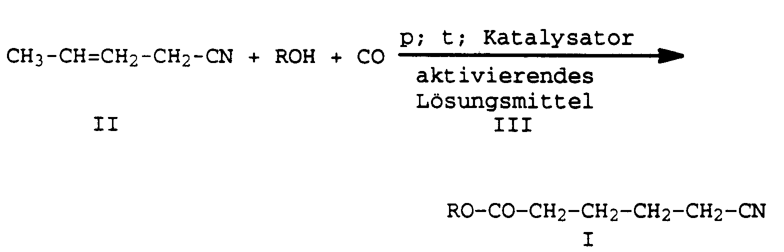 R = Alkyl

Anstelle des Cyanbut-2-ens können auch das 1-en- und das 3-en-Isomere oder auch Isomerengemische eingesetzt werden, denn unter den Reaktionsbedingungen stellt sich ein Isomerengleichgewicht ein. Weiterhin eignen sich sowohl die trans- als auch die cis-Isomeren des Cyanbut-1-ens und des Cyanbut-2-ens als Ausgangsverbindungen für das erfindungsgemäße Verfahren. Aus wirtschaftlichen Gründen setzt man bevorzugt das Cyanbut-2-en ein, welches durch Addition von Cyanwasserstoff an Butadien leicht zugänglich ist.

Das gute Gelingen des erfindungsgemäßen Verfahrens ist von der Art der Alkanole ROH (II) grundsätzlich nicht abhängig. In Betracht kommen daher C₁- bis C₁₂-Alkanole, darunter bevorzugt C₁- bis C₄-Alkanole sowie vor allem Methanol und Ethanol.

Pro Mol des 1-Cyanbutens setzt man in der Regel 0,5 bis 10, vorzugsweise 1 bis 5 mol des Alkanols II ein. Unterstöchiometrische Mengen an Alkanol können sich empfehlen, wenn der Umsatz des Cyanbutens begrenzt werden soll, um beispielsweise die Selektivität bezüglich des gewünschten Verfahrensproduktes (I) zu erhöhen.

Wesentlich für das erfindungsgemäße Verfahren ist die Mitverwendung eines der definitionsgemäßen aktivierenden Lösungsmittel IIIa bis IIIc,

R-O-CO-O-R (IIIa)

R₂N-CO-O-R (IIIb)

R₂N-CO-NR₂ (IIIc)

in denen die Reste R gleich oder verschieden sein können und für C₁- bis C₆-Alkylgruppen oder C₅- bis C₇-Cycloalkylgruppen stehen, wobei diese Reste auch unter Ausbildung 5- bis 7-gliedriger Ringe miteinander verbunden sein können.

Als Kohlensäurediester IIIa kommen vorzugsweise symmetrische Ester in Betracht, wie Kohlensäuredimethylester und Kohlensäurediethylester. Besonders bevorzugt sind cyclische Kohlensäureester wie Ethylencarbonat, 1,2-Propylencarbonat, 1,3-Propylencarbonat und 1,2-Isobutylencarbonat.

Geeignete Carbaminsäureester IIIb sind vorzugsweise N,N-Dimethylcarbaminsäurebutylester und N,N-Diechylcarbaminsäurebutylester sowie vor allem cyclische Ester wie 3-Methyl-2-oxazolidinon, 3-Ethyl-2-oxazolidinon, 3-Propyl-2-oxazolidinon, 3-Butyl-2-oxazolidinon, 3-Pentyl-2-oxazolidinon, 3,4-Dimethyl-2-oxazolidinon, 3,5-Dimethyl-2-oxazolidinon, 3-Methyl-4-ethyl-2-oxazolidinon und 3-Ethyl-5-methyl-2-oxazolidinon.

Als Harnstoffe IIIc empfehlen sich cyclische Harnstoffderivate wie N,N'-Dimethylethylenharnstoff, N,N'-Diethylethylenharnstoff und N,N'-Dimethylpropylenharnstoff sowie vor allem offenkettige Verbindungen wie N,N,N',N'-Tetramethylharnstoff, N,N,N',N'-Tetraethylharnstoff.

Die Menge des aktivierenden Lösungsmittels oder auch von Gemischen dieser aktivierenden Lösungsmittel kann in der Regel frei gewählt werden. Bevorzugt verwendet man möglichst geringe Mengen. Die Anfangskonzentration der aktivierenden Lösungsmittel in der gesamten Reaktionsmischung kann 20 bis 85 Gew.-%, vorzugsweise 30 bis 75 Gew.-%, besonders bevorzugt 35 bis 65 Gew.-% betragen.

Oberhalb des genannten Bereiches erzielt man zwar zumeist gute Selektivitäten bezüglich der geradkettigen 5-Cyanvaleriansäureester, dafür aber sinkt die Raum-Zeit-Ausbeute der Umsetzung, und der Aufwand für die Aufarbeitung nimmt zu.

Die Reaktion kann auch in Gegenwart weiterer organischer Lösungsmittel durchgeführt werden, sofern diese unter den Reaktionsbedingungen inert sind. Zum Beispiel eignen sich Kohlenwasserstoffe wie n-Pentan, n-Hexan, Toluol und die Xylole, daneben Ether, z.B. Tetrahydrofuran, Dioxan oder Ethylenglykoldimethylether, sowie Carbonsäureester wie Methylacetat, Ethylacetat und Butylacetat.

Im übrigen nimmt man die Carbonylierung in an sich bekannter Weise vor, also bei Temperaturen von 130 bis 200 °C, vorzugsweise 140 bis 190 °C und bei einem Druck von 50 bis 700 bar, vorzugsweise 100 bis 350 bar.

Für die Umsetzung wird bevorzugt reines Kohlenmonoxid verwendet, jedoch kann ein Gehalt an Wasserstoff, welcher kleiner als 10 Vol.-% sein sollte, je nach Ausführungsform des erfindungsgemäßen Verfahrens auch zur Aktivierung des Cobaltkatalysators führen.

Als Cobaltkatalysatoren eignen sich Dicobaltoctacarbonyl oder Cobaltcarbonylwasserstoff.

Anstelle dieser Carbonylkomplexe kann man auch Salze wie Cobaltacetat, Cobaltformiat oder Cobalt-2-ethylhexanoat einsetzen, vor allem aber die Cobaltsalze derjenigen Carbonsäuren, die bei der Reaktion untergeordnet als Nebenprodukte entstehen können, wie beispielsweise Cobaltvalerat, Cobaltadipat oder Cobaltmethylglutarat. Aus diesen Salzen bilden sich die aktiven Komplexe unter den Reaktionsbedingungen in situ.

Die Konzentration der Katalysatoren beträgt in der Regel 0,1 bis 5, vorzugsweise 0,3 bis 3 Gew-% Cobalt, bezogen auf die Gesamtmenge des Reaktionsgemisches.

Die Bildung einer katalytisch wirksamen Cobaltverbindung kann durch Zugabe von Wasser beschleunigt werden, wobei die Menge des Wassers vorzugsweise 0,5 bis 4 mol pro Mol Cobalt beträgt.

Man kann das Verfahren nach den hierfür bekannten Techniken kontinuierlich oder diskontinuierlich ausführen.

Zur Erzielung befriedigender Selektivitäten bezüglich des Verfahrensproduktes I führt man die Reaktion bevorzugt bis zu einem Umsatz von 35 bis 70 % bezogen auf das 1-Cyanbuten durch.

Die Aufarbeitung des Reaktionsgemisches erfolgt in an sich bekannter Weise, und zwar vorzugsweise durch fraktionierte Destillation. Die hierbei anfallenden Lösungsmittel und Ausgangsverbindungen werden zweckmäßigerweise in die Carbonylierung zurückgeführt.

Man erhält die linearen Cyanoester, bezogen auf 50%-igen Umsatz des 1-Cyanbutens, in der Regel in Selektivitäten von 80 bis 90 %. Daneben entstehen noch etwa 0,5 bis 5 %, vor allem der sich von den Alkanolen II ableitenden Adipinsäuredialkylester, und 0,5 bis 8 % Valeronitril sowie geringe Mengen sonstiger Nebenprodukte.

Das erfindungsgemäße Verfahren hat den Vorteil, daß auch bei Verwendung relativ kleiner Mengen der besagten aktivierenden Lösungsmittel IIIa bis IIIc überraschend hohe Selektivitäten bezüglich der geradkettigen 5-Cyanvaleriansäureester (I) erzielt werden.

Die nach dem erfindungsgemäßen Verfahren hergestellten Ester der 5-Cyanvaleriansäure sind Vorstufen für das bei der Herstellung von Polyamiden bedeutsame ε-Caprolactam.

### Beispiele

### Beispiel 1

Ein Autoklav mit einem Volumen von 107 ml wurde mit einer Lösung aus
- 27,2: Gew.-% 1-Cyanbut-2-en
- 21,2: Gew.-% Methanol
- 48,2: Gew.-% N,N,N',N'-Tetramethylharnstoff
- 2,8: Gew.-% Co₂(CO)₈ und
- 0,6: Gew.-% Wasser
gefüllt. Anschließend wurden bei einem CO-Druck von rund 200 bar und 160°C stündlich 10,3 g der gleichen Lösung und 1,72 Nl Kohlenmonoxid-Gas kontinuierlich zugeführt und der kontinuierlich entnommene Austrag gaschromatographisch analysiert (interner Standard Benzonitril).

Folgende Ergebnisse wurden erzielt (GC):

| | |
|---|---|
| Umsatz des Cyanbutens: | 47 % |
| Selektivität zu Cyanestern: | 89 % |
| - davon zu I (n-Anteil): | 98 % |

Der Austrag wurde wie üblich destillativ aufgearbeitet.

### Beispiel 2

Die Versuchdurchführung erfolgte analog Beispiel 1, wobei pro Stunde 8,9 g einer Lösung aus
- 27,2: Gew.-% 1-Cyanbut-2-en
- 21,2: Gew.-% Methanol
- 48,2: Gew.-% N,N'-Dimethylpropylenharnstoff
- 2,8: Gew.-% Co₂(CO)₈ und
- 0,6: Gew.-% Wasser
mit 1,72 Nl Kohlenmonoxid-Gas umgesetzt wurden. Folgende Ergebnisse wurden erzielt (GC):

| | |
|---|---|
| Umsatz des Cyanbutens: | 39 % |
| Selektivität zu Cyanestern: | 89 % |
| - davon zu I (n-Anteil): | 96 % |

### Beispiel 3

Die Versuchdurchführung erfolgte analog Beispiel 1, wobei pro Stunde 9,6 g einer Lösung aus
- 27: Gew.-% 1-Cyanbut-2-en
- 21,3: Gew.-% Methanol
- 48,4: Gew.-% 3-Methyl-2-oxazolidinon
- 2,8: Gew.-% Co₂(CO)₈ und
- 0,5: Gew.-% Wasser
mit 1,68 Nl Kohlenmonoxid-Gas umgesetzt wurden. Folgende Ergebnisse wurden erzielt (GC):

| | |
|---|---|
| Umsatz des Cyanbutens: | 57 % |
| Selektivität zu Cyanestern: | 93 % |
| - davon zu I (n-Anteil): | 96 % |

### Beispiel 4

Die Versuchdurchführung erfolgte analog Beispiel 1, wobei pro Stunde 8,8 g einer Lösung aus
- 16: Gew.-% 1-Cyanbut-2-en
- 12,5: Gew.-% Methanol
- 69,5: Gew.-% Propylencarbonat
- 1,7: Gew.-% Co₂(CO)₈ und
- 0,3: Gew.-% Wasser
mit 1,68 Nl Kohlenmonoxid-Gas umgesetzt wurden. Folgende Ergebnisse wurden erzielt (GC):

| | |
|---|---|
| Umsatz des Cyanbutens: | 63 % |
| Selektivität zu Cyanestern: | 93 % |
| - davon zu I (n-Anteil): | 95 % |

### Beispiel 5

Auf 120 g einer Lösung aus
- 27: Gew.-% 1-Cyanbut-2-en
- 21,3: Gew.-% Methanol
- 48,4: Gew.-% 3-Butyl-2-oxazolidinon
- 2,8: Gew.-% Co₂(CO)₈ und
- 0,5: Gew.-% Wasser
wurden in einem Autoklaven 100 bar CO aufgepreßt und danach die Reaktionsmischung auf 160°C erhitzt. Der Druck wurde anschließend durch Nachpressen von Kohlenmonoxid auf 210 bar erhöht und 6 Stunden bei 160°C gehalten. Der Austrag wurde wie oben gaschromatographisch analysiert.

Folgende Ergebnisse wurden erzielt (GC):

| | |
|---|---|
| Umsatz des Cyanbutens: | 44 % |
| Selektivität zu Cyanestern: | 88 % |
| - davon zu I (n-Anteil): | 94 % |

Der Austrag wurde danach wie üblich destillativ aufgearbeitet.

### Beispiel 6

Der Versuch wurde wie in Beispiel 5 beschrieben durchgeführt. Die Reaktionsdauer betrug 4 Stunden.

Zum Einsatz kamen 120 g einer Lösung aus
- 27: Gew.-% 1-Cyanbut-2-en
- 21,3: Gew.-% Methanol
- 48,4: Gew.-% 3-Methyl-2-oxazolidinon
- 2,8: Gew.-% Co₂(CO)₈ und
- 0,5: Gew.-% Wasser.

Folgende Ergebnisse wurden erzielt (GC):

| | |
|---|---|
| Umsatz des Cyanbutens: | 66 % |
| Selektivität zu Cyanestern: | 90 % |
| - davon zu I (n-Anteil): | 96 % |

### Beispiel 7

320 g einer Lösung aus
- 27,2: Gew.-% 1-Cyanbut-2-en
- 21,2: Gew.-% Methanol
- 48,2: Gew.-% 3-Methyl-2-oxazolidinon
- 2,8: Gew.-% Co₂(CO)₈ und
- 0,6: Gew.-% Wasser
wurden bei 160°C und einem CO-Druck von 30 bar einem Autoklaven zugeführt und dort bei der gleichen Temperatur und unter einem CO-Druck von 270 bar 4 Stunden der Carbonylierungsreaktion unterworfen. Der Austrag wurde wie oben gaschromatographisch analysiert.

Folgende Ergebnisse wurden erzielt (GC):

| | |
|---|---|
| Umsatz des Cyanbutens: | 53 % |
| Selektivität zu Cyanestern: | 90 % |
| - davon zu I (n-Anteil): | 96 % |

## Patentansprüche

1. Verfahren zur Herstellung von 5-Cyanvaleriansäureestern (I) durch Carbonylierung eines 1-Cyanbutens mit Kohlenmonoxid und einem dem Esterrest entsprechenden Alkanol (II) bei erhöhter Temperatur und erhöhtem Druck in Gegenwart eines Cobaltkatalysators sowie in Gegenwart eines aktivierenden Lösungsmittels, dadurch gekennzeichnet, daß man als aktivierendes Lösungsmittel wirksame Mengen eines Kohlensäurediesters, eines Carbaminsäureesters oder eines Harnstoffes der allgemeinen Formeln IIIa, IIIb bzw. IIIc
R-O-CO-O-R (IIIa)
R₂N-CO-O-R (IIIb)
R₂N-CO-NR₂ (IIIc)
oder von Gemischen dieser Verbindungen verwendet, in denen die Reste R gleich oder verschieden sein können und für C₁- bis C₆-Alkylgruppen oder C₅- bis C₇-Cycloalkylgruppen stehen, wobei diese Reste auch unter Ausbildung 5- bis 7-gliedriger Ringe miteinander verbunden sein können.

2. Verfahren nach Anspruch 1, dadurch gekennzeichnet, daß man als aktivierendes Lösungsmittel IIIa einen cyclischen Kohlensäureester mit 5- bis 7-gliedrigen Ringen verwendet.

3. Verfahren nach Anspruch 1, dadurch gekennzeichnet, daß man als aktivierendes Lösungsmittel IIIb einen cyclischen Carbaminsäureester mit 5 bis 7 Ringgliedern verwendet, der am Stickstoffatom eine C₁- bis C₆-Alkylgruppe trägt und am Ring bis zu 2 C₁- bis C₂-Alkylgruppen tragen kann.

4. Verfahren nach Anspruch 1, dadurch gekennzeichnet, daß man als aktivierendes Lösungsmittel IIIc einen Harnstoff verwendet, dessen Reste R gleich oder verschieden sein können und für C₁- bis C₆-Alkylgruppen stehen, oder in dem zwei Reste R eine die beiden N-Atome verbindende Ethylen- oder 1,2-Propylen- oder 1,3-Propylengruppe und die übrigen Reste R Methyl oder Ethyl bedeuten.

5. Verfahren nach den Ansprüchen 1 bis 4, dadurch gekennzeichnet, daß die Anfangskonzentration der aktivierenden Lösungsmittel IIIa, IIIb bzw. IIIc im Reaktionsgemisch 35 bis 65 Gew.-% beträgt.

6. Verfahren nach den Ansprüchen 1 bis 5, dadurch gekennzeichnet, daß man als Alkanol (II) Methanol oder Ethanol verwendet.

## Claims

1. A process for preparing 5-cyanovaleric esters (I) by carbonylation of a 1-cyanobutene with carbon monoxide and an alkanol (II) corresponding to the ester radical at elevated temperature and elevated pressure in the presence of a cobalt catalyst and also in the presence of an activating solvent, characterized in that the activating solvent used comprises effective amounts of a carbonic diester, of a carbamic ester or of a urea of the general formulae IIIa, IIIb and IIIc
R-O-CO-O-R (IIIa)
R₂N-CO-O-R (IIIb)
R₂N-CO-NR₂ (IIIc)
where the radicals R can be identical or different and represent C₁-C₆-alkyl groups or C₅-C₇-cycloalkyl groups, and these radicals can also be linked to one another to form 5- to 7-membered rings, or of mixtures of these compounds.

2. A process as claimed in claim 1, characterized in that a cyclic carbonic ester with 5- to 7-membered rings is used as activating solvent IIIa.

3. A process as claimed in claim 1, characterized in that activating solvent IIIb used is a cyclic carbamic ester having 5 to 7 ring members which carries a C₁-C₆-alkyl group on the nitrogen atom and can carry up to 2 C₁-C₂-alkyl groups on the ring.

4. A process as claimed in claim 1, characterized in that activating solvent IIIc used is a urea whose radicals R can be identical or different and represent C₁-C₆-alkyl groups, or in which two radicals R denote an ethylene or 1,2-propylene or 1,3-propylene group linking the two nitrogen atoms and the other radicals R are methyl or ethyl.

5. A process as claimed in claims 1 to 4, characterized in that the starting concentration of activating solvents IIIa, IIIb and IIIc in the reaction mixture is from 35 to 65% by weight.

6. A process as claimed in claims 1 to 5, characterized in that methanol or ethanol is used as alkanol (II).

## Revendications

1. Procédé de préparation d'esters de l'acide 5-cyanovalérique (I) par carbonylation d'un 1-cyanobutène avec du monoxyde de carbone et un alcanol (II) correspondant au résidu d'ester à température élevée et à pression élevée en présence d'un catalyseur au cobalt, de même qu'en présence d'un solvant d'activation, caractérisé en ce qu'on utilise à titre de solvant d'activation des quantités efficaces d'un diester d'acide carbonique, d'un ester d'acide carbamique ou d'une urée répondant aux formules générales IIIa, IIIb, respectivement IIIc
R-O-CO-O-R (IIIa)
R₂N-CO-O-R (IIIb)
R₂N-CO-NR₂ (IIIc)
ou de mélanges de ces composés, dans lesquels les résidus R peuvent être identiques ou différents et représentent des groupes alkyles en C₁ à C₆ ou des groupes cycloalkyles en C₅ à C₇, ces résidus pouvant également être reliés ensemble en formant des cycles de 5 à 7 membres.

2. Procédé selon la revendication 1, caractérisé en ce qu'on utilise à titre de solvant d'activation IIIa un ester cyclique d'acide carbonique avec des cycles de 5 à 7 membres.

3. Procédé selon la revendication 1, caractérisé en ce qu'on utilise à titre de solvant d'activation IIIb un ester cyclique d'acide carbamique de 5 à 7 membres de cycle qui porte un groupe alkyle en C₁ à C₆ sur l'atome d'azote et qui peut porter jusqu'à deux groupes alkyles en C₁ à C₂ sur le cycle.

4. Procédé selon la revendication 1, caractérisé en ce qu'on utilise à titre de solvant d'activation IIIc une urée dont les résidus R peuvent être identiques ou différents et représentent des groupes alkyles en C₁ à C₆ ou dans laquelle deux résidus R signifient un groupe d'éthylène ou de 1,2-propylène ou de 1,3-propylène, reliant les deux atomes d'azote, et les autres résidus R signifient un groupe méthyle ou éthyle.

5. Procédé selon les revendications 1 à 4, caractérisé en ce que la concentration initiale des solvants d'activation IIIa, IIIb, respectivement IIIc, s'élève à 35 à 65 % en poids dans le mélange réactionnel.

6. Procédé selon les revendications 1 à 5, caractérisé en ce qu'on utilise à titre d'alcanol (II) du méthanol ou de l'éthanol.
